# FASCICULE DE BREVET EUROPEEN

(11) **EP 0 672 394 B1**
(45) Date de publication et mention de la délivrance du brevet: **28.07.1999**
(21) Numéro de dépôt: 95500010.4
(22) Date de dépôt: 08.02.1995
(51) Int. Cl.: A61F 2/02, A61F 2/06, A61M 25/01

(54) **Prothèse urétérale de longue durée**
Harnleiterprothese für einen langfristigen Einsatz
Long duration ureteral prosthesis

(30) Priorité: 25.02.1994 ES 9400384
(43) Date de publication de la demande: 20.09.1995
(73) Titulaire: Izquierdo de la Torre, Fernando, E-08029 Barcelona (ES)
(72) Inventeur: Izquierdo de la Torre, Fernando, E-08029 Barcelona (ES)
(74) Mandataire: Espiell Volart, Eduardo Maria

(56) Documents cités:
- EP-A- 0 418 381
- EP-A- 0 516 189
- FR-A- 2 512 678
- US-A- 4 813 925
- US-A- 4 820 262

## Description

La présente invention concerne une prothèse urétérale de celles du type extractible, dont les caractéristiques essentielles permettent sa simple implantation ainsi que sa longue durée, en pouvant rester à l'intérieur de l'organisme pour des périodes de temps même supérieures à un an, en permettant, tel qu'il est indiqué, sa facile extraction, ainsi que son remplacement par une autre aux mêmes caractéristiques.

Les techniques endo-urologiques complémentaires, d'usage normal et actuellement très fréquent, comme peuvent être les manoeuvres de soutien préalables au traitement, ainsi que les manoeuvres nécessaires pour résoudre les complications qui pourraient se présenter, rendent souvent nécessaire l'usage de prothèses urétérales, qui doivent être placées à l'intérieur de l'organisme.

L'objet de ces prothèses est d'obtenir la circulation normale de l'urine depuis le rein jusqu'à la vessie urinaire dans les situations où les processus obstructifs pathologiques l'empêchent. Par ailleurs, l'objet de cette prothèse est de faciliter l'évacuation et l'élimination de calculs formés dans les reins, soit s'ils sont expulsés selon les méthodes connues, sans fragmenter le calcul, soit s'ils sont fragmentés auparavant par le biais d'une des techniques modernes actuellement utilisées. La pose de la prothèse, dans les deux cas, permettra une évacuation facile et peu douloureuse.

La perméabilité des parois de la prothèse qu'il faut implanter doit permettre, à tous moments, de maintenir et de conserver la dynamique péristaltique normale de la voie urinaire.

La prothèse urétérale motif de la présente invention, est dessinée pour être placée à l'intérieur de la voie urinaire, de sorte qu'elle la parcourt depuis le bassinet jusqu'à la vessie urinaire à travers le conduit de l'uretère.

La pose dont il a été question, dans la voie urinaire, se fait généralement au moyen de manoeuvres endoscopiques ou de procédés percutanés antérogrades ou rétrogrades.

Il faut absolument que ladite prothèse soit dessinée et fabriquée de sorte qu'elle puisse rester très longtemps à l'intérieur de l'organisme sans perdre ses propriétés intrinsèques, des périodes de temps qui, pour la commodité du patient et l'efficacité du traitement, devraient être, même, de plus d'un an.

Les propres caractéristiques de la prothèse doivent permettre sa facile extraction par des procédés endoscopiques, permettant ainsi son rapide remplacement par une autre aux mêmes caractéristiques.

Certaines caractéristiques basiques de ce type de prothèses urétérales ont été détaillées, elles sont suffisamment appliquées dans la prothèse objet de la présente invention.

Ainsi, donc, et en précisant quelles sont les caractéristiques essentielles de ces types de prothèse et en argumentant les essentialités de la prothèse objet de cette invention, on peut résumer ainsi:

La prothèse doit être facilement insérée au moyen de quelque modalité technique, comme peuvent être lesdites manoeuvres endoscopiques, les procédés percutanés antérogrades ou rétrogrades.

Il faut qu'elle reste toute située complètement à l'intérieur, ce qui arrive dans l'agencement de la prothèse décrit dans ce brevet, qui occupe la totalité de l'uretère, depuis le bassinet jusqu'à la vessie urinaire.

Il faut qu'elle soit dessinée de sorte que toute possibilité de déplacement spontané soit éliminée, ce qui est garanti au moyen des extrémités préformées en forme de crosse et avec mémoire pour la récupération de cet agencement, en étant, de plus, parcourue à l'intérieur par un fil de fer métallique fixé aux deux extrémités et qui empêche toutes possibilités d'allongement élastique que pourraient provoquer les manoeuvres confrontées de traction.

Il faut que cette prothèse soit facilement extractible ou remplaçable, une caractéristique qu'elle possède également, car ladite opération peut se faire par le procédé simplement endoscopique.

Les matériaux employés pour son élaboration, doivent être opaques aux radiations pour permettre son observation par radiographie; biologiquement inertes ou biotolérables et chimiquement stables, indispensable pour rester à l'intérieur de l'organisme sans provoquer de rejet et ne pas être attaqués par les liquides provenant du rein.

La présente invention a pour objet de remedier aux inconvenients précités.En effet, aucune prothèse urétrale n'est connue qui serait métallique complète, ni expansible; extractible, à parois perméables pour l'unire, mais filtrant le passage de fragments de calcul et ne permettant pas l'entrée de la muqueuse urétrale lors de sa croissance.

La fabrication de la prothèse objet de la présente invention en fil de fer d'acier inoxydable d'alliage approprié, permet de remplir plus qu'il ne faut les conditions requises qui caractérisent une prothèse de qualité. Ces matériaux empêcheront également l'incrustation de sédiments.

Le bon flux urinaire est garanti para la caractéristique de construction basique de la prothèse, car l'enroulement du fil de fer pour former la paroi de la prothèse lui confère une perméabilité élevée, en conservant ainsi la dynamique péristaltique normale de la voie urinaire.

Le matériau employé permet de conférer à l'ensemble une bonne mémoire de retenue en sa forme et agencement, notamment à ses extrémités en forme de crosse, tandis qu'il permet, finalement, d'offrir un coût raisonnable de fabrication, avec un excellent rapport coût-qualité, en tenant compte de l'utilisation sanitaire du produit.

Les affections pour lesquelles l'usage de la prothèse en double crosse, c'est-à-dire en forme de crosse à chaque extrémité, est très indiqué est principalement, l'obstruction accompagnée d'inflammation de l'uretère, l'anurie obstructive, l'obstruction aiguë, la colique néphrétique réfractaire au traitement chimiolithique, lithiase obstructive chez les femmes enceintes et iatrogènie en chirurgie endoscopique.

Pour tout ce qui a été exposé et en considérant les prothèses connues et uitlisées jusqu'à présent, il faut remarquer les complications qui peuvent surgir avec une prothèse à double crosse installée et dont les caractéristiques ne seraient pas tout à fait les justes: infection urinaire-septique, obstruction, incrustations, position anormale, perforation et rupture, érosion, hématurie intense, nouage spontané, ce dernier étant un inconvénient très peu fréquent.

En faisant un bref résumé de l'histoire de la technique en ce qui concerne ces types de prothèses, il faut citer le début de l'utilisation de prothèse spécifiquement dans l'uretère, au début de ce siècle, par Albarran; cependant, pendant les dernières années de nouveaux modèles ont surgis, fabriqués avec des matériaux plus résistants et inertes.

En 1967, D.D. ZIMSKIND et ses collaborateurs ont créé un cathéter auto-fixable pour le drainage prolongé de l'urine, inséré cystoscopiquement, qui avait le défaut principal de la facilité de son émigration ou déplacement involontaire.

R.P. GIBBONS a présenté une prothèse en silicone ayant de multiples barbes ou reliefs, afin de garantir sa fixation, mais qui donnait pour résultat une prothèse au diamètre excessif.

D.L. MAC CULLOUGH et T.N. HEPERLEN ont introduit les extrémités souples en forme de "J" pour l'auto-fixation de la prothèse.

En 1978, R.P. FINNEY a fabriqué les premières prothèses avec les deux extrémités en "J" semblables aux actuelles, et il faut se rappeler que déjà en 1907 POUSSON a créé la première fixation en"J".

Le brevet US-A-4813925 de Anderson Jr. et al. consiste en un tuyau creux qui est utilisé pour infuser un liquide à son intérieur afin de faire descendre les calculs et ses spires son discontinues. De plus, comme il s'agit d'une prothèse autoexpansible, elle comprime la paroi de l'uretère pour obtenir que les calculs descendent à son intérieur, ce qui peut produire des lésions précisément au patient.

En ce qui concerne les matériaux actuellement employés, il faut indiquer de façon plus précise que le matériau influe en grande partie sur les propriétés physiques et la biocompatibilité de la prothèse; de là son importance.

Actuellement, les matériaux les plus employés ont été les polymères de silicone, les polyuréthannes, le silitec, l'uro-soft et le c-flex, mais, dans la pratique continue, ils ont montré des problèmes sérieux, comme, par exemple, la silicone, qui, de par sa souplesse présente de grandes difficultés lors de son installation; l'uro-soft, très fragile; le polyuréthanne, plus résistant mais son opacité aux radiations est faible, pour ne pas dire nulle. De plus, le fait que ces prothèses doivent être perforées font que les orifices se bouchent facilement et de là leur manque de commodité.

Cest plus récemment qu'on est arrivé à une conclusion importante, basique pour l'application pratique de ces prothèses, lorsqu'on a déterminé que les calculs ne se collent pas à l'acier, ce qui arrive avec la matière plastique et d'autres matières organiques.

Cest ainsi que l'exprime C.Y.C.PAK, de "Center for Mineral Metabolism" de la Southwestern Medical School de l'Université de Dallas, au Texas, Etats Unis, dans son ouvrage intitulé "Physicochemical action and extrarrenal manifestations of Alkali therapy, 1988" et dans celui intitulé "Urologic research" de VR Walker, Ral Sutton et collaborateurs, de Plenum Press, New York, 1989.

Cela montre l'importance de la caractéristique essentielle de la prothèse objet de ce brevet d'invention, fabriquée en acier, tel que cela sera décrit plus loin.

Finalement et en faisant référence à la méthodologie d'implantation actuellement utilisée, il faut citer qu'elle est faite par la voie rétrograde, de préférence, soit à travers un cystoscope, un urétéroscope, un cathéter urétéral ou par voie ouverte; cela se fait également par voie antérograde, à travers un néphroscope, un urétéroscope, la chirurgie ouverte ou la néphrostonie percutanée.

Les modalités techniques actuelles sont habituellement:

La méthode endoscopique simple, la méthode endoscopique radiologique, la méthode de reconversion de cathéter urétéral simple ou celle de la chirurgie ouverte.

Aucune prothèse urétrale n'a été décrite qui serait métallique, complète, non expansible, extractible, à parois perméables pour l'urine, mais filtrant le passage des fragments de calcul et ne permettant pas l'entrée de la muqueuse urétrale lors de sa croissance.

L'objectif principal de l'invention est d'obtenir une structure tubulaire métallique flexible à parois perméables et aux extrémités non agressives pour la voie urinaire. Les extrémités non agressives ont la forme de J et il faut que ce J soit élastique, c'est-à-dire, que sa forme puisse être rectifiée pour pouvoir être placé et retiré en récupérant immédiatement après sa forme initiale.

Les extrémités ont la forme de J pour éviter la probable lésion des reins ou de la vessie comme ferait une extrémité droite. Elles rendent le passage de l'urine aisée à pression faible à leur intérieur. De même, elles rendent plus aisée l'immobilisation de la prothèse dans la voie urinaire.

La prothèse urétérale de longue durée, objet du présent brevet (voir revendication 1) , présente comme caractéristiques essentielles qu'elle est constituée par une structure tubulaire, formée par un fil de fer métallique enroulé en spirale, qui constitue la paroi de ladite prothèse.

Les spires de fil de fer métallique enroulé viennent en contact chacune, étroitement avec la précédente et avec la suivante, en formant ainsi un tuyau souple mais à paroi perméable dans les deux sens aux liquides, dans ce cas à l'urine. La longueur et le calibre du tuyau ainsi constitué est fixe et ne peuvent être modifiés; dans le cas où il faudrait une longueur ou un calibre différent, il faudra appliquer un modèle différent.

Les deux extrémités de la prothèse tubulaire hélicoïdale sont préformées en forme de crosse et avec la mémoire qui lui permettra de récupérer immédiatement et toujours cette conformation spéciale aux deux extrémités.

Cela permet l'introduction rectiligne de la prothèse par le conduit urinaire, en récupérant la forme d'extrémité en crosse au moment où les deux extrémités restent déjà définitivement situées dans le bassinet et dans la vessie urinaire.

L'intérieur de la prothèse décrite est parcouru par un fil de fer métallique très fin, qui ne diminue pratiquement pas le passage libre de la prothèse, qui restera fixée de façon appropriée, généralement par soudure, aux deux extrémités de la prothèse et par ses dernières spires de l'hélicoïde, et qui empêchera l'allongement élastique de la prothèse dans sa longueur, suite aux manoeuvres confrontées de traction auxquelles ladite prothèse pourrait être soumise.

Dans le processus opérationnel de pose de la prothèse, on utilisera comme accessoire ultérieurement extractible, une tige métallique très longue, d'environ un mètre et demi, introduite à l'intérieur de ladite prothèse, qui maintiendra l'ensemble droit, en facilitant sa pose. Cette tige possèdera une extrémité souple, c'est-à-dire, celle qui est introduite à l'intérieur du corps du patient pour éviter de blesser l'intérieur des organes par où elle va passer.

Une fois la prothèse est posée, la tige tel comme il a été dit, ayant une longueur très supérieure à celle de la prothèse, sera facilement retirée, une opération qui se fera immédiatement, en tirant de l'extrémité qui reste à l'extérieur du patient. La prothèse étant ainsi située, et étant données les caractéristiques essentielles déjà décrites aussi bien du matériau de fabrication que de sa forme et construction, la prothèse maintiendra parfaitement ouverte la totalité du conduit urinaire, dont les parois organiques resteront en contact avec la surface extérieure de la prothèse, en permettant, étant donnée son élasticité, le passage de l'urine vers l'intérieur de la prothèse, en s'écoulant le long du tuyau qu'elle configure, tandis que les calculs le feront par l'extérieur de la prothèse, en évitant toutes possibilités d'obstruction de celle-ci.

Afin de pouvoir décrire en détails les éléments qui constituent la prothèse urétérale objet de la présente invention, il est annexé à la présente description des dessins dans lesquels, comme exemple non limitatif, il a été représenté une mise en oeuvre pratique de la prothèse citée.

Dans ces dessins:
La figure 1 dessine schématiquement et en coupe partielle, la prothèse de l'invention en illustrant parfaitement le fil de fer intérieur fixé à celle-ci et les cambrures aux deux extrémités en forme de crosse;
La figure 2 est un détail, également à une plus grande échelle, de l'extrémité ou embouchure de la prothèse, montrant la fixation par soudure du fil de fer intérieur sur les trois ou quatre spires finales et qui évite l'allongement de la prothèse.
La figure 3 est un détail, à une plus grande échelle, d'une des extrémités de la prothèse pliée en forme de crosse.
La figure 4 est une vue partielle des deux extrémités de la prothèse, lorsqu'on a introduit en son intérieur la tige auxiliaire et elle a adopté la position rectiligne, optimale pour l'introduction dans l'organisme du patient.
La figure 5 est une vue schématique qui montre le mode d'introduction, à l'aide de la tige de guide, de la prothèse qui occupera, finalement, le bassinet, la vessie de l'urine et l'uretère; et
La figure 6 est une vue semblable à la précédente mais qui montre la prothèse déjà posée et où elle a déjà adopté les formes de crosse aux deux extrémités.

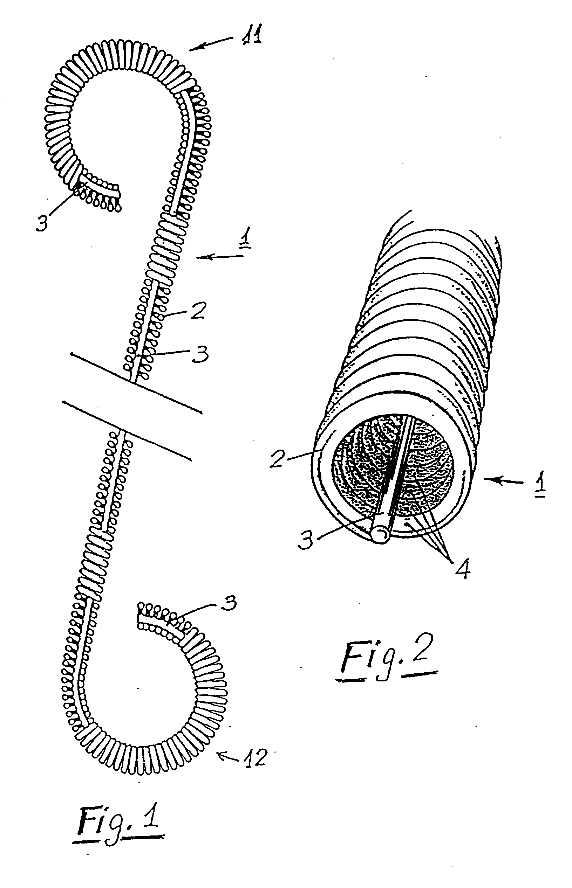

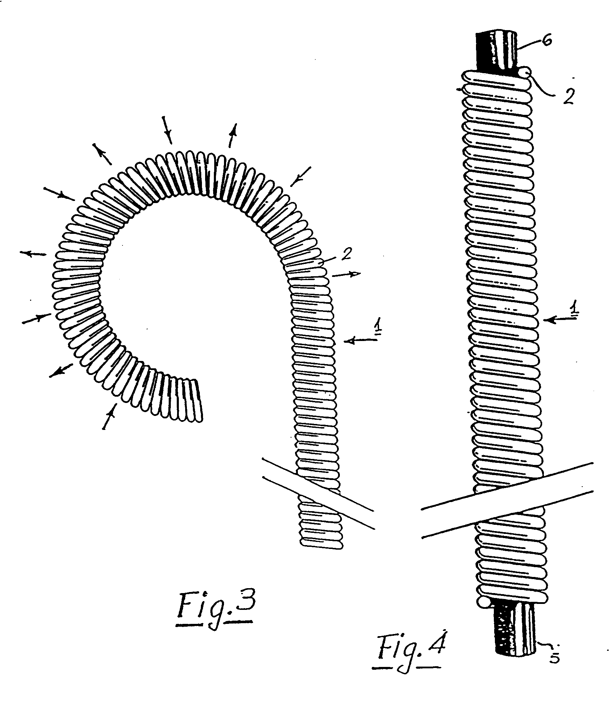

Selon les dessins, la prothèse urétérale de longue durée, objet de cette invention, est constituée par la structure tubulaire (1), formée par le fil de fer métallique inoxydable et biotolérable (2), enroulé en spirale, dont les spires viennent en contact les unes avec les autres, comme il est noté aux figures 1, 2, 3 et 4, en permettant, néanmoins, le passage de l'urine, du dedans vers le dehors et vice versa, tel qu'indiquent les flèches de la figure 3 et, plus spécialement, dans l'ouverture qu'offrent les spires dans les zones en forme de crosse.

A l'intérieur de ladite structure tubulaire (1) reste agencé le fil de fer (3), dont les extrémités sont soudées à la face interne des spires des extrémités (4) de la prothèse, tel que l'on note clairement dans la figure 2.

A l'intérieur de la prothèse, lorsqu'il faudra qu'elle soit posée à l'intérieur du conduit urinaire, on situera la tige (5) très longue, qui maintient droite la prothèse et qui est pourvue à une extrémité, précisément celle qui sera introduite à l'intérieur du patient, d'une zone (6) en matériau souple qui évitera agresser par où elle passera et blesser ainsi l'organisme.

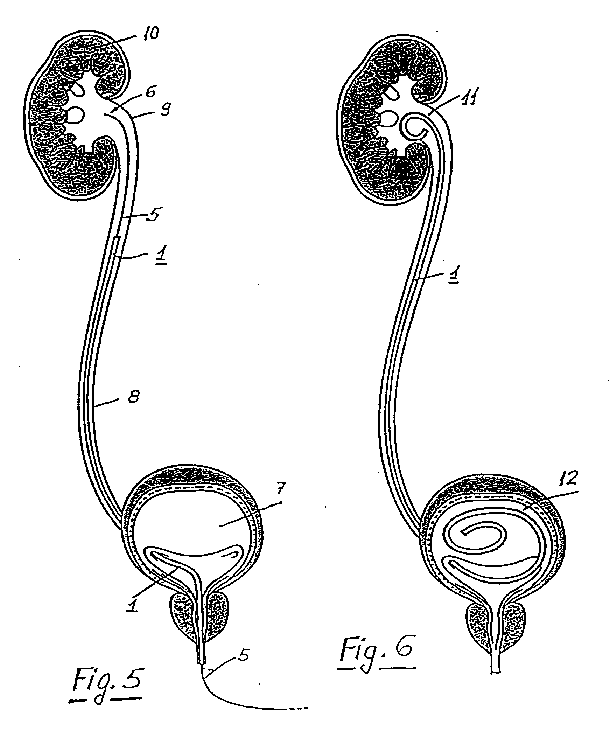

La prothèse, tel que représente la figure 5, sera posée depuis la vessie urinaire (7) et à travers le conduit urinaire (8), jusqu'au bassinet (9) et le rein (10).

La prothèse ainsi posée permettra la circulation normale de l'urine depuis le rein (10) jusqu'à la vessie (7), lorsque un processus pathologique l'empêcherait au cas où ladite prothèse ne serait pas utilisée. Tel qu'on a déjà dit, l'urine pourra circuler à l'intérieur de la structure tubulaire (1), tandis que les possibles calculs le feront entre ladite structure (1) et le conduit urinaire (8).

La stabilité et l'amovibilité de la prothèse (1) sont complètement garanties par l'enroulement de ses deux extrémités en forme de crosse (11) et (12), une fois situées à l'intérieur du rein (10) et de la vessie (7), respectivement, tel que dessine la figure 6, position permanente que forme ladite prothèse, comme montre la figure 1, seulement perdue au moment de la pose, par l'action de la tige intérieure auxiliaire de guide (5), et immédiatement récupérée dès que la tige est retirée.

Le passage de l'urine par la prothèse de l'invention est très facile et cela parce que les calculs ou les dépôts propres de l'urine ne peuvent pas se coller au matériau et qu'il n'y a pas non plus d'orifices de part en part, comme c'est le cas de prothèses construites en matière plastique, qui se bouchent très facilement et dont la récupération est impossible. L'urine passe de dedans au dehors ou vice versa sans obstacles et plus précisément dans les parties qui adoptent la forme de crosse car dans la zone externe il se produit des arcs de séparation plus accusés.

Suffisamment décrites les diverses caractéristiques essentielles de cette invention, il faut indiquer que les tailles, l'aspect intérieur et les variations qui n'altèrent pas l'essentialité des formes et de qualité des matériaux employés, n'altèreront pas l'essentialité de cette invention, qui est résumée dans les revendications suivantes.

## Revendications

1. Prothèse urétérale extractible de longue durée constituée par une structure tubulaire creuse (1) formée par un premier fil de fer métallique biotolérable (2), enroulé en spirale, les spires du premier fil de fer étant agencées de sorte que chacune vienne étroitement en contact avec les spires contigues, caractérisée en ce que la structure tubulaire dispose à son intérieur et le long de toute la structure tubulaire creuse (1) un deuxième fil de fer métallique (3) fin, qui est fixé à la face interne sur les trois ou quatre dernières spires (4) des deux extrémités, empêchant ainisi l'allongement élastique de la prothèse, et grâce au deuxième fil de fer métallique interne (3), la structure tubulaire a ses extrémités préformés en forme de crosse (11 et 12) avec la suffisante mémoire pour toujours permettre d'inmédiatement récupérer cet agencement spécial, notamment lorsque les deux extrémités restent déjà définitivement disposées dans le bassinet (9) et dans la vessie urinaire (7) du patient, permetant le passage facile de l'urine au niveau des ouvertures qu'offrent les spires dans la zone en forme de crosse dans les deux sens, c'est-à-dire, de l'exterieur à l'interieur et viceversa.

2. Prothèse urétérale extractible de longue durée, selon la revendication précedénte, caractérisée en ce que le deuxième fil de fer est soudé à la face interne de la structure tubulaire.

3. Prothèse urétérale extractible de longue durée, selon les revendications précédents, caractérisée en ce qu'elle est dotée d'une tige métallique accessoire (5) plus longue que la prothèse (1) et sur laquelle passe, au moment de sa pose, la structure tubulaire (2) de ladite prothèse, dont l'extrémité à introduire présente une zone souple et qui ne blesse pas, et qui maintendra ladite structure rectiligne durant le processus de son introduction, en étant facilement extractible lorsque le processus finit.

## Claims

1. Extractable long-lasting urethral prosthesis constituted by a hollow tubular structure (1) formed by a first biotolerable metal wire (2), spiral wound, the spires of the first wire being arranged so that each comes closely in contact with contiguous spires, characterized in that the tubular structure has available within it and along the whole hollow tubular structure (1) a second thin metal wire (3) which is attached to the internal face on the three or four last spires (4) of the two ends, preventing thus the prosthesis elastic elongation and thanks to a second internal metal wire (3) the tubular structure at its ends crook-shaped pre-formed (11 and 12) with sufficient memory for always allowing to immediately recover this special arrangement, namely when the two ends already remain definitely arranged in the patient's pelvis (9) and urinary bladder (7), allowing the urine to easily passes at the level of the openings provided by the spires in the crook-shaped area in both senses, i.e., from outside inwardly and vice versa.

2. Extractable long-lasting urethral prosthesis, according to above claim, characterized in that the second wire is welded to the internal face of the tubular structure.

3. Extractable long-lasting urethral prosthesis, according to above claims, characterized in that it is provided with an accessory metal rod (5) longer than the prosthesis (1) and on which passes, at the moment of the assembly, the tubular structure (2) of said prosthesis, whose end to be introduced shows a flexible area which does not hurt and which will keep this structure rectilinear during the process of its introduction being easily extractable when the process is completed.

## Patentansprüche

1. Herausnehmbare Harnröhrenprothese mit langer Lebensdauer, die aus einer Hohlröhrenstruktur (1) besteht, gebildet durch einen ersten bio-verträglichen Metalldraht (2), der spiralförmig aufgerollt ist, wobei die Windungen des ersten Drahts so aufgerollt ist, wobei die Windungen des ersten Drahts so angerichtet sind, daß jede einzelne Windung mit den nachfolgenden Windungen in engem Kontakt steht, dadurch gekennzeichnet, daß die Röhrenstruktur in ihrem Inneren und an der ganzen Hohlröhrenstruktur (1) entlang über einen zweiten feinen Metalldraht (3) verfügt, der an der inneren Seite an den drei oder vier letzten Windungen (4) der beiden Enden befestigt ist, womit die elastische Verlängerung der Prothese vermieden wird und dank des zweiten internen Metalldrahts (3), der kreuzförmig an den beiden Enden befestigt ist (11) und (12) und über einen ausreichenden Speicher verfügt, um in jedem Moment diese spezielle Anordnung wiederherzustellen, insbesondere dann, wenn beide Enden bereits definitiv im Becken (9) und in der Harnblase (7) des Patienten disponiert sind, den ungehemmten Ablauf des Urins an den Öffnungen der kreuzförmigen Windungen der Zone in beiden Richtungen, d.h. von außen nach innen und umgekehrt ermöglicht.

2. Herausnehmbare Harnröhrenprothese mit langer Lebensdauer, gemäß des vorangegangenen Patentanspruchs, dadurch gekennzeichnet, daß der zweite Draht an der inneren Seite der Röhrenstruktur angeschweißt ist.

3. Herausnehmbare Harnröhrenprothese mit langer Lebendauer, gemäß der beiden vorangegangenen Patentansprüche, dadurch gekennzeichnet, weil diese über einen zusätzlichen Metallstab (5) verfügt, der länger als die Prothese (1) selbst ist und, im Moment der Anbringung, darüber hinausragt, und weil die Röhrenstruktur (2) der Prothese, deren einzuführendes Ende flexibel ist und keine Verletzungen verursacht sowie diese Struktur während des Einführungsprozesses gerade hält, leicht herausgenommen werden kann, wenn der Vorgang beendet ist.
